# EUROPÄISCHE PATENTSCHRIFT

(11) **EP 2 596 763 B1**
(45) Veröffentlichungstag und Bekanntmachung des Hinweises auf die Patenterteilung: **08.01.2014**
(21) Anmeldenummer: 11190843.0
(22) Anmeldetag: 25.11.2011
(51) Int. Cl.: A61F 2/46

(54) **Werkzeug zum Setzen oder Entfernen von Gelenkkomponenten künstlicher Gelenkpfannen, insbesondere für Hüftgelenkendoprothesen**
Tool for setting or removing joint components of artificial joint sockets, in particular for hip joint prostheses
Outil de fixation ou de suppression de composants articulés de rotules artificielles, notamment pour endoprothèses de la hanche

(43) Veröffentlichungstag der Anmeldung: 29.05.2013
(73) Patentinhaber: Waldemar Link GmbH & Co. KG, 22339 Hamburg (DE)
(72) Erfinder: Dmuschewsky, Klaus, 22397 Hamburg (DE)
(74) Vertreter: Raffay & Fleck

(56) Entgegenhaltungen:
- EP-A1- 1 438 936
- WO-A1-2005/122970
- DE-A1- 10 250 390
- FR-A1- 2 948 013
- US-A1- 2006 149 285

## Beschreibung

Die vorliegende Erfindung betrifft ein Werkzeug zum Setzen oder Entfernen von Gelenkkomponenten künstlicher Gelenkpfannen, insbesondere für Hüftgelenkendoprothesen gemäß den Merkmalen des Oberbegriffs des Anspruches 1. Sie betrifft ferner ein Set zur bedarfsweisen Bildung eines solchen Werkzeuges.

Im Rahmen der orthopädischen Chirurgie können heute für verschiedene Gelenke bei Funktionsstörungen z.B. aufgrund von Verschleiß oder aufgrund von das Gelenk betreffenden Erkrankungen wie beispielsweise Tumorerkrankungen die natürlichen Gelenke durch künstliche Gelenke bzw. Gelenkendoprothesen ersetzt werden. Dies betrifft auch solche Gelenke, die Gelenkpfannen aufweisen, in denen Gelenkköpfe verdrehbar gelagert sind. Hierbei ist insbesondere das Hüftgelenk zu nennen, bei welchem der Einsatz bzw. Einbau entsprechender Hüftendogelenkprothesen heutzutage routinemäßig vorgenommen wird.

Am Beispiel solcher Hüftgelenkendoprothesen wird bei deren Versorgung neben einem künstlichen Gelenkkopf am proximalen Femurende im Bereich des Beckenknochens die natürliche Gelenkpfanne entfernt und durch eine künstliche Gelenkpfanne ersetzt. Diese Gelenkpfanne setzt sich in der Regel zusammen aus einer einen Pfannenhohlraum aufweisenden Gelenkkomponente, in deren Pfannenhohlraum dann ein Einsatz eingesetzt wird, welcher die eigentliche Gelenkpfanne bildet und der häufig aus keramischen Material oder aber Kunststoff besteht. Dabei wird die erstgenannte, den Pfannenhohlraum aufweisende Gelenkkomponente im Beckenknochen fest verankert, was auf unterschiedliche Weise gesehen kann. So gibt es Formen, in denen mit Knochenzement gearbeitet wird, gleichermaßen gibt es zementfrei zu setzende Gelenkkomponenten. Grob unterschieden wird hierbei auch zwischen solchen Gelenkkomponenten, die eingeschlagen werden und durch einen reinen Presssitz halten, und solchen, die mit einem Außengewinde versehen selbstschneidend in den Beckenknochen geschraubt werden.

Für die angestrebte Funktionalität des nachzubildenden Gelenkes ist eine den ursprünglichen anatomischen Verhältnissen so weit als möglich nachempfundene Ausrichtung und entsprechend gerichtete Verankerung der einzelnen Gelenkkomponenten in den Knochen von höchster Wichtigkeit. Entsprechend müssen die Gelenkkomponenten, hierbei insbesondere die im Beckenknochen zu verankernden, den Pfannenhohlraum aufweisenden Gelenkkomponenten sehr genau gehandhabt und ausgerichtet werden können. Zugleich müssen für die Verankerung im Knochen hohe Kräfte aufgebracht werden.

Um dieses zu bewerkstelligen, werden entsprechende Instrumente zum Setzen der Gelenkkomponente verwendet. Vergleichbare Instrumente kommen ggf. auch zum Einsatz, um eine einmal gesetzte Gelenkkomponente, beispielsweise im Zuge einer Revisionsoperation, wieder zu entfernen.

Gattungsgemäße Werkzeuge zum Setzen bzw. Entfernen von solchen Gelenkkomponenten sind z.B. in der DE 10 2007 020 484 A1 und der DE 60 2005 004 890 T2 offenbart und beschrieben. Derartige Werkzeuge weisen einen Werkzeugschaft auf, der an seinem distalen Ende einen Halteabschnitt aufweist zum Festlegen der den Pfannenhohlraum aufweisenden Gelenkkomponente. An einem zweiten, proximalen Ende ist ein Griffabschnitt angeordnet. Da die den Pfannenhohlraum aufweisende Gelenkkomponente nicht an ihrer Außenseite gehandhabt werden kann, da diese Außenseite im Verlauf der Operation in dem Knochen zu verankern ist, muss an dieser Gelenkkomponente von innen her, also in dem Pfannenhohlraum angegriffen und diese dort festgelegt werden.

Bei heute bekannten Werkzeugen geschieht dies sehr häufig durch einen am distalen Ende des Halteabschnittes angeordneten Gewindezapfen, der mit einer im Bereich des Pols der Gelenkkomponente eingebrachten Gewindebohrung zusammenwirkt. Zum Verbinden der Gelenkkomponente mit dem Werkzeug wird diese mit der Gewindebohrung einfach auf den Gewindezapfen aufgeschraubt. Diese Art der Festlegung ist in der genannten DE 10 2007 020 484 A1 offenbart. Problematisch bei dieser Art der Verbindung ist insbesondere, dass im Falle von mittels Presssitz im Knochen zu verankernden Gelenkkomponenten, die eingeschlagen werden müssen, die in Längsrichtung des Gewindezapfens aufgebrachten Kräfte zu einer Verformung der Gewinde führen können, was im schlimmsten Falle ein Ablösen des Werkzeuges nach Einbringen der Gelenkkomponente in den Knochen verhindert, so dass die gerade implantierte Gelenkkomponente wieder entfernt werden muss. Abgesehen davon, dass solche Vorkommnisse meldepflichtige Vorkommnisse sind, bedeutet dies für den Patienten einen schwereren Eingriff, da in einem solchen Fall typischerweise bereits während der Erstversorgung eine Revision vorzunehmen ist, innerhalb derer z.B. bei einer Hüftgelenksoperation der Beckenknochen im Implantationsbereich weiter zu reduzieren und eine Gelenkkomponente größerer Dimension zu verwenden ist.

Auch eignen sich derartige Verbindungen für solche Instrumente, mit denen die Gelenkkomponenten durch Einschrauben mit dem Knochen verbunden werden, lediglich für eine der Schraubrichtungen, nämlich entweder das Einschrauben oder das Ausschrauben, d.h. das Entfernen einer solchen Gelenkkomponente aus dem Knochen. Denn nur in einer der Drehrichtungen, also in einem Schraubsinn, kann hier eine Übertragung der Kräfte bzw. Drehmomente stattfinden, in der jeweils anderen Schraubrichtung würde sich das Werkzeug von der Gelenkkomponente lösen, ohne dass Kraft auf die Verbindung zwischen der Gelenkkomponente und dem Knochen übertragen werden kann.

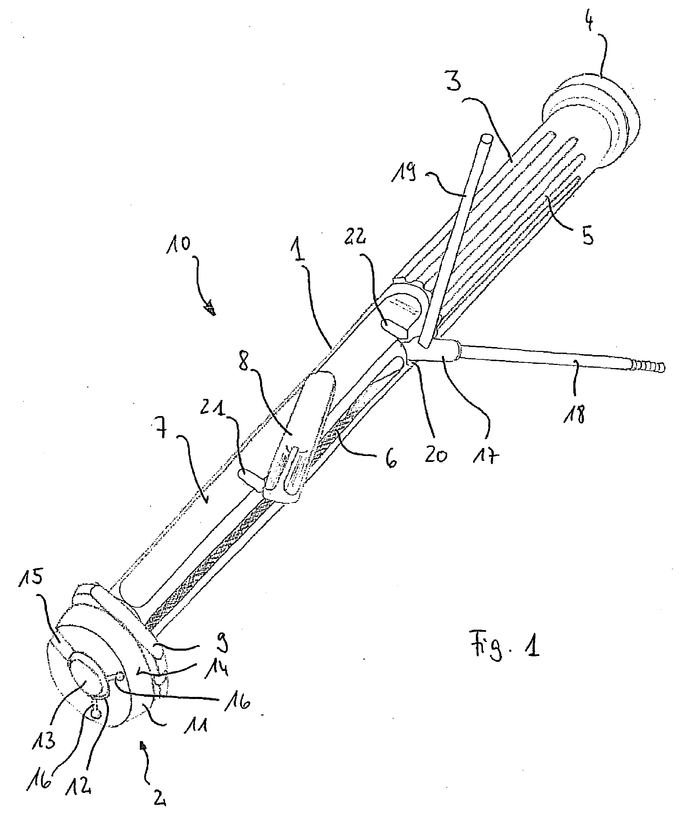

In der DE 60 2005 004 890 T2 ist ein anderes Werkzeug vorgeschlagen, welches die Gelenkkomponente vermittels eines anzulegenden Unterdruckes in dem Halteabschnitt halten soll. Nachteilig bei dieser Lösung ist, dass eine zusätzliche Komponente mit dem Werkzeug zu verbinden ist, nämlich eine Vakuumpumpe, die über einen Schlauch an den in Fig. 1 dieser Entgegenhaltung mit 50 bezeichneten Zapfen anzuschließen ist. Ferner besteht die Gefahr, dass unter der Operation Partikel in das Kanalsystem des Vakuumkanals eindringen, dieses blockieren und so ein Lösen des Instrumentes von der Gelenkkomponente nach Implantation erschweren oder gar unmöglich machen.

Der nächstliegende Stand der Technik wird in DE-A-102 50 390 offenbart.

Aufgabe der vorliegenden Erfindung ist es daher, ein gattungsgemäßes Werkzeug dahingehend weiterzubilden, dass eine feste und zuverlässige Verbindung der zu implantierenden Gelenkkomponente an dem Halteabschnitt ermöglicht wird, die sich ebenso zuverlässig wieder lösen lässt. Dabei soll diese Verbindungstechnik sowohl für solche Gelenkkomponenten verwendbar sein, die durch Aufbringen axialer Press- bzw. Schlagkräfte in den Knochen eingeschlagen werden, als auch bei solchen, die durch eine selbstschneidende Gewindeverbindung in den Knochen eingeschraubt werden. Zudem soll das Werkzeug in der angestrebten Weiterentwicklung universell und für die unterschiedlichsten Typen von Gelenkkomponenten künstlicher Gelenkpfannen verwendbar sein, insbesondere soll für diesen Zweck ein Set zur bedarfsweisen Bildung eines entsprechenden Werkzeuges angegeben werden.

Die Lösung der oben genannten Aufgabe in ihren unterschiedlichen Aspekten besteht erfindungsgemäß in einem Werkzeug zum Setzen oder Entfernen von Gelenkkomponenten künstlicher Gelenkpfannen, insbesondere für Hüftgelenkendoprothesen, mit den Merkmalen des Anspruches 1. Der oben angesprochene Aspekt der zur Verfügung Stellung eines flexiblen Satzes bzw. Sets zur bedarfsweisen Bildung eines Werkzeuges ist mit einem solchen Set aufweisend die Merkmale des Anspruches 11 berücksichtigt. Vorteilhafte Weiterbildungen des erfindungsgemäßen Werkzeuges sind in den abhängigen Ansprüchen 2 bis 10, eine Weiterbildung des Sets ist in Anspruch 12 angegeben.

Erfindungsgemäß weist das Werkzeug zum Setzen oder Entfernen von Gelenkkomponenten künstlicher Gelenkpfannen, insbesondere für Hüftgelenkendoprothesen einen langgestreckten Schaft auf. Dieser hat an einem ersten, distalen Ende einen Halteabschnitt zum Festlegen einer einen Pfannenhohlraum aufweisenden Gelenkkomponente. Im Bereich eines zweiten, proximalen Endes des Schaftes ist ein Griffabschnitt angeordnet. In dem erfindungsgemäßen Werkzeug weist der Halteabschnitt ein in dem Pfannenhohlraum einführbares, radial nach außen spreizbares Klemmmittel sowie ein mit dem Klemmmittel zusammenwirkendes Spreizmittel zum nach außen Spreizen des Klemmmittels auf.

Durch diese Gestaltung des erfindungsgemäßen Werkzeuges in seinem Halteabschnitt wird ein Festhalten einer einen Pfannenhohlraum aufweisenden Gelenkkomponente mittels Klemmkräften ermöglicht. Das Klemmmittel, welches radial nach außen spreizbar ist, kann somit im Inneren des Pfannenhohlraumes gegen dessen Wandung gedrückt werden und so entweder durch reine Klemmkraft ein Festhalten der Gelenkkomponente besorgen oder aber auch unterstützt durch einen Formschluss, wenn beispielsweise in dem Pfannenhohlraum ein Hinterschnitt oder eine ähnliche Struktur gebildet ist, in den bzw. die entsprechend ausgebildete Gegenstrukturen auf der radial außen liegenden Oberfläche des Klemmmittels eingreifen können.

Diese Art der Festlegung der Gelenkkomponente an dem Halteabschnitt des erfindungsgemäßen Werkzeuges bietet verschiedene Vorteile. So wird verglichen mit der Verbindung über eine rein an der Polregion der Gelenkkomponente angeordnete Gewindebohrung eine zuverlässigere und für eine Kraftübertragung besser geeignete Verbindung erreicht, die sich breitflächiger, insbesondere über eine polauswärts gelegene Oberflächenregion des Pfannenhohlraumes erstreckt und insoweit besonders haltesicher ist. Auch treten hier, wenn das Werkzeug für eine Einschlaggelenkkomponente verwendet wird, nicht die Probleme auf, die es bei einer Gewindeverbindung geben kann, dass nämlich beim Aufbringen von axialen Kräften das Gewinde verformen und schlimmstenfalls festgesetzt werden kann. Gleichermaßen ist diese Art der Festlegung der Gelenkkomponente an dem Halteabschnitt aber auch für solche Gelenkkomponenten geeignet, die in den Knochen einzuschrauben sind. Um hier die rotatorisch wirkenden Kräfte bzw. Drehmomente aufzunehmen und auf die Gelenkkomponente zu übertragen, können mit Vorteil entsprechende Mitnehmerstrukturen, beispielsweise ein Stift, Steg oder dgl. Vorsprung bzw. ein korrespondierender Rücksprung an dem Werkzeug vorgesehen sein zum Zusammenwirken mit der jeweils korrespondierend ausgebildeten Strukturen an der Gelenkkomponente.

Im Vergleich zu der weiteren aus dem Stand der Technik bekannten Lösung, bei der mittels eines angelegten Unterdruckes die Gelenkkomponente im Halteabschnitt gehalten wird, bietet die erfindungsgemäße Lösung eine Vereinfachung dadurch, als dass dieses Werkzeug autark betrieben werden kann, ohne dass es während des Gebrauches mit weiteren Komponenten, insbesondere einer Unterdruck erzeugenden Pumpe bzw. Vakuumpumpe verbunden werden muss. Eine solche für das bekannte Instrument erforderliche Verbindung behindert den Operateur in seiner Bewegungsfreiheit, so dass das Überwinden dieses Erfordernisses einen erheblichen Vorteil darstellt.

Mit Vorteil kann das Spreizmittel zum Spreizen bzw. Lösen des Klemmmittels in Richtung des Verlaufes des Schaftes bewegbar sein. Dabei kann das Spreizmittel ein Zugmittel aufweisen, welches sich über zumindest einen Teil der Länge des Schaftes, vorzugsweise im Inneren desselben, erstreckt. Als ein solches Zugmittel kommen grundsätzlich flexible Mittel wie etwa ein Seil oder ein Zugdraht in Betracht, allerdings werden stangenähnliche Zugmittel bevorzugt, die jedenfalls quer zu ihrer Längsrichtung bzw. zum Längsverlauf des Schaftes im Wesentlichen starr sind.

Das Klemmmittel weist mit Vorteil ein elastisches Element auf, welches eine zum distalen Ende hin sich erweiternd verlaufende Öffnung aufweist. Dabei hat das Spreizmittel dann einen in der Öffnung des elastischen Elementes sitzenden, in Richtung des Griffabschnittes, also des proximalen Endes des Schaftes bewegbaren Spannkörper mit korrespondierender, sich in Richtung des proximalen Endes verjüngender Außenkontur. Eine besonders einfache Kombination solcher Strukturen sind konische Formen, also eine konische Öffnung in dem elastischen Element, die sich in Richtung des proximalen Endes des Schaftes verjüngt und ein entsprechender Spannkonus mit korrespondierend gebildeter Außenkontur, die sich ebenfalls in Richtung des proximalen Endes hin verjüngt. Eine solche Kombination ermöglicht ein einfaches Spreizen des elastischen Elementes durch Ziehen des Spannkörpers in Richtung des proximalen Endes des Schaftes. Das elastische Element, welches seine Spreizfähigkeit und Elastizität mit Vorteil insbesondere durch wenigstens einen in seiner Umfangsrichtung an wenigstens einer Position vorgesehenen Schlitz erhält bzw. erhöht, ggf. aber auch mehrere solcher Schlitze, wobei im Falle des Vorsehens mehrerer Schlitze mit Vorteil lediglich einer der Schlitze durchgehend die Außenwandung des elastischen Elementes durchbricht, kann beispielsweise und insbesondere aus Kunststoff gebildet sein, ohne auf dieses Material beschränkt zu sein. Mit Vorteil wird ein solches Material gewählt, welches hinsichtlich seiner Oberflächeneigenschaften mit dem Material auf der Innenseite des Pfannenhohlraums der Gelenkkomponente bei angelegter Spannkraft eine Materialkombination von hohem Reibwiderstand und damit besonders guter Haltekraft bildet.

Mit Vorteil weist das Klemmmittel ferner eine am distalen Ende des Schaftes angeordnete Anlageplatte als Gegenlager für das elastische Element auf. Diese Anlageplatte kann dabei mit Vorteil auch zusätzlich eine Anlagestruktur für den Rand der den Pfannenhohlraum aufweisenden Gelenkkomponente bilden. Diese Anlageplatte dient somit jedenfalls als Gegenlager bzw. Widerlager für das elastische Element, gegen welches es beim Bewegung des Spannkörpers in Richtung des proximalen Endes des Schaftes gezogen wird, so dass der Spannkörper in der Öffnung des elastischen Elementes verschoben wird und dieses durch das Zusammenwirken der jeweils komplementär verlaufenden Oberflächen auseinanderdrückt und spreizt. Die zusätzlich mögliche und bevorzugte Eigenschaft der Anlageplatte als Anlage für einen Rand der den Pfannenhohlraum aufweisenden Gelenkkomponente, bringt insbesondere den Vorteil mit sich, dass für die Verwendung des Werkzeuges mit einzuschlagenden Gelenkkomponenten die axialen Einschlagkräfte über den Rand der Komponente aufgebracht werden und damit eine bessere Verteilung erfahren, als wenn diese, wie insbesondere im Falle von einschraubten Gelenkkomponenten häufig der Fall, allein über die Polregion der Gelenkkomponente aufgebracht werden. Im Falle der Verwendung des Werkzeuges für einzuschraubende Gelenkkomponenten können im Bereich der Anlage der Platte am Rand der den Pfannenhohlraum aufweisenden Gelenkkomponente Mitnahmestrukturen wie Vorsprünge, Stifte, Stege, Rücksprünge oder dgl. angeordnet sein, die mit entsprechenden Gegenstrukturen an dem Rand der Gelenkkomponente zusammenwirken zum Übertragen des mittels des Werkzeuges aufzubringenden Drehmomentes.

Die Anlageplatte ist mit Vorteil so dimensioniert und gestaltet, dass sie in Abschnitten eine geringere Ausdehnung aufweist als die Ausdehnung der Gelenkkomponente, die darauf anzuordnen ist, so dass sie den Operateur einen Blick auf die proximale Kante bzw. den Rand der Gelenkkomponente erlaubt.

Mit Vorteil weist die Anlageplatte eine Öffnung auf, in welcher das elastische Element mit einem einen Hinterschnitt aufweisenden Halteabschnitt lösbar festlegbar ist. Diese Art der konstruktiven Gestaltung erlaubt eine einfache Festlegung des elastischen Elementes an der Anlageplatte, wobei insbesondere der Halteabschnitt um die Öffnung des elastischen Elementes herum gebildet sein kann, so dass bei in diese Öffnung eingesetztem Spannkörper das elastische Element nicht mehr in einer Weise verformt werden kann, dass der Halteabschnitt von der Anlageplatte zu lösen ist.

Eine nicht beanspruchte Lösung, nach der das Zugmittel ein quer zur Längsrichtung des Schaftes im Wesentlichen starres Element ist, welches um einen von dem distalen Ende in Richtung des proximalen Endes versetzt gelegenen Lagerpunkt verschwenkbar an dem Schaft festgelegt ist und an seinem distalen Ende die Anlageplatte in einer Öffnung durchragt, und wobei das distale Ende des Schaftes und das Spreizmittel und/oder das Klemmmittel so ausgebildet ist/sind, dass durch unter Überwindung einer Rückhaltekraft vorgenommenes Verschwenken des Zugmittels die Anlageplatte über das distale Ende des Schaftes hinweggeschoben und so das Klemmmittel von dem distalen Ende des Schaftes gelöst werden bzw. in umgekehrter Richtung an dem distalen Ende des Schaftes angeordnet und festgelegt werden kann, erlaubt ein besonders einfaches und schnelles Wechseln des Klemmmittels. Dies kann beispielsweise auch unter einer Operation erforderlich sein, wenn erst im Verlaufe der Operation festgestellt werden kann, welches Klemmmittel ein für die Entfernung einer Gelenkkomponente geeignetes ist.

Für ein besonders einfaches Wechseln des Klemmmittels ist es von Vorteil, wenn in der Anlageplatte und dem elastischen Element durchgehende Schlitze vorgesehen sind, mittels derer die genannten Elemente über das Zugmittel führbar und so auf dieses aufsetzbar bzw. von diesem lösbar sind. Die Rückhaltekraft, die beim Verschwenken des Zugmittels zum Lösen der Anlageplatte und des elastischen Elementes von dem distalen Ende des Schaftes zu überwinden ist, kann insbesondere eine von dem elastischen Element aufgebrachte Klemmkraft sein. Dadurch, dass sich beim Verschwenken über das distale Ende des Schaftes hinweg zunächst eine Entfernung der Anlageplatte von dem Schwenkpunkt ergibt, bis die Anlageplatte über den Rand des distalen Endes des Schaftes geführt ist, ergibt sich eine relative Verlagerung zwischen dem elastischen Element und dem Spreizmittel, wodurch letzteres ein Spreizen des elastischen Elementes besorgt, dieses also entgegen der in Richtung der Grundstellung wirkenden elastischen Kraft auslenkt. Um hier eine zusätzliche Federkraft zu erzielen, ist erfindungsgemäß das Zugmittel in Längsrichtung des Schaftes elastisch aus gebildet. So kann auch durch die Elastizität des Zugmittels die Rückhaltekraft aufgebracht bzw. unterstützt werden. Eine Elastizität des Zugmittels ist aber auch für andere Lösungen gemäß der Erfindung von Vorteil, insbesondere kann sie eine Kraft, mit der das Klemmmittel gespreizt wird, begrenzen, um mögliche Beschädigungen zu vermeiden. Übersteigt nämlich die Spreizkraft, die auf das Klemmmittel aufgebracht wird, die Kraft, die erforderlich ist, das Zugmittel in Längsrichtung zu dehnen, so erfolgt diese Dehnbewegung, und die Kräfte werden hierüber abgeleitet. Dabei ist das Zugmittel eine Federzugstange. Diese kann insbesondere S-förmig oder sogar doppelt S-förmig sein. Eine solche Federzugstange hat den Vorteil, dass sie in ihrer Längsrichtung elastisch, in Richtung quer dazu jedoch vergleichsweise starr gebildet ist.

Zum Bewegen des Zugmittels und damit zum Betätigen des Spreizmittels und nach außen Spreizen des Klemmmittels kann an dem Schaft ein Exzenterhebel angeordnet sein, auf dem ein proximales Ende des Zugmittels in exzentrischer Lagerung angeordnet ist. Dabei weist dieser Exzenterhebel mit Vorteil einen Totpunkt auf, bei dessen Überschreiten er das Zugmittel in einer Stellung verriegelt, in der das Spreizmittel das Klemmmittel radial nach außen spreizt. So kann gesichert und verriegelt eine Stellung des Werkzeuges gehalten werden, in welchem die Gelenkkomponente in dem Halteabschnitt sicher und fest gehalten ist.

Das erfindungsgemäße Werkzeug besteht bevorzugt aus nur einigen und mit einfachen und wenigen Handgriffen zu zerlegenden Einzelkomponenten, so dass für eine Reinigung und Sterilisation des Werkzeuges nach erfolgtem Einsatz im Zuge einer Operation nur wenige Handgriffe erforderlich sind.

Ferner kann ein Peilmittel, beispielsweise in Form eines Peilstabes oder eines gegabelten Peilstabpaares vorgesehen sein, welches an dem Schaft des Werkzeuges in vorgegebener Stellung befestigbar ist und dem Operateur ein Ausrichten der Gelenkkomponente zum Setzen erleichtert.

Wie bereits erwähnt besteht ein Aspekt der Erfindung auch darin, ein Set anzugeben, mit dem ein Werkzeug bedarfsweise gebildet werden kann. Dieses Set umfasst erfindungsgemäß wenigstens einen Schaft mit einem Spreizmittel und zumindest zwei auswechselbar an dem distalen Ende des Schaftes anordbare Klemmmittel unterschiedlicher Geometrie. Damit kann aus einem solchen Set mit einfachen und schnellen Handgriffen ein spezifisches Werkzeug für das Setzen bzw. Entfernen bestimmter Gelenkkomponenten gebildet werden. Abhängig von der Gelenkkomponente und ihrer Geometrie wird das entsprechende Klemmmittel passender Geometrie gewählt. Wie für das Werkzeug an sich der Schaft eine unterschiedliche Form aufweisen kann, beispielsweise vollständig gerade und in Flucht verlaufend, einfach gekröpft mit versetzter Längsachse, doppelt gekröpft oder dgl., so kann auch das Set eine entsprechende Auswahl unterschiedlich geformter Schäfte aufweisen, um auch hier passend für den jeweiligen Einsatzzweck eine entsprechend Schaftform auswählen zu können.

Weitere Vorteile und Merkmale der Erfindung ergeben sich aus der nachfolgenden Beschreibung eines Ausführungsbeispiels anhand der beigefügten Figuren. Dabei zeigen:
- Fig. 1: ein Ausführungsbeispiel eines erfindungsgemäßen Werkzeuges zum Setzen oder Entfernen von Gelenkkomponenten künstlicher Gelenkpfannen in einer dreidimensionalen Ansicht;
- Fig. 2: das Werkzeug aus Fig. 1 in einer teilweise geschnittenen Darstellung;
- Fig. 3: vergrößert einige der für die Umsetzung der Spreizkräfte wesentliche Komponenten des Werkzeuges gemäß Fig. 1;
- Fig. 4: das erfindungsgemäße Werkzeug mit einzelnen Teilen in einer Explosionsdarstellung;
- Fig. 5: in zwei Ansichten a und b Kombinationen aus jeweils unterschiedlich gestalteten elastischen Elementen und Anlageplatten für verschiedene Ausbildungen der Klemmmittel;
- Fig. 6: schematisch und geschnitten drei unterschiedliche Ansichten zur Verdeutlichung der lösbaren Festlegung des Zugmittels am Exzenterhebel;
- Fig. 7: in vier verschiedenen Darstellungen einzelschrittweise schematisch das Vorgehen zum Verbinden des elastischen Elementes mit der Anlageplatte;
- Fig. 8: in zwei Darstellungen a und b schematisch zwei unterschiedliche Klemmmittel bestehend aus verschiedenen elastischen Elementen und Anlageplatten;
- Fig. 9: schematische Darstellungen zur Verdeutlichung der Funktionsweise des Verbindens des Klemmmittels mit dem Zugmittel und Anordnen desselben am distalen Ende des Schaftes;
- Fig. 10: in vier verschiedenen Ansichten weitere Detaildarstellungen einzelner Elemente des Werkzeuges;
- Fig. 1: eine zweite Variante eines erfindungsgemäßen Werkzeuges, hier ausgeführt als Werkzeug zum Ein- bzw. Ausschrauben einer Gelenkkomponente in den Knochen bzw. aus diesem heraus in zwei Darstellungen a und b;

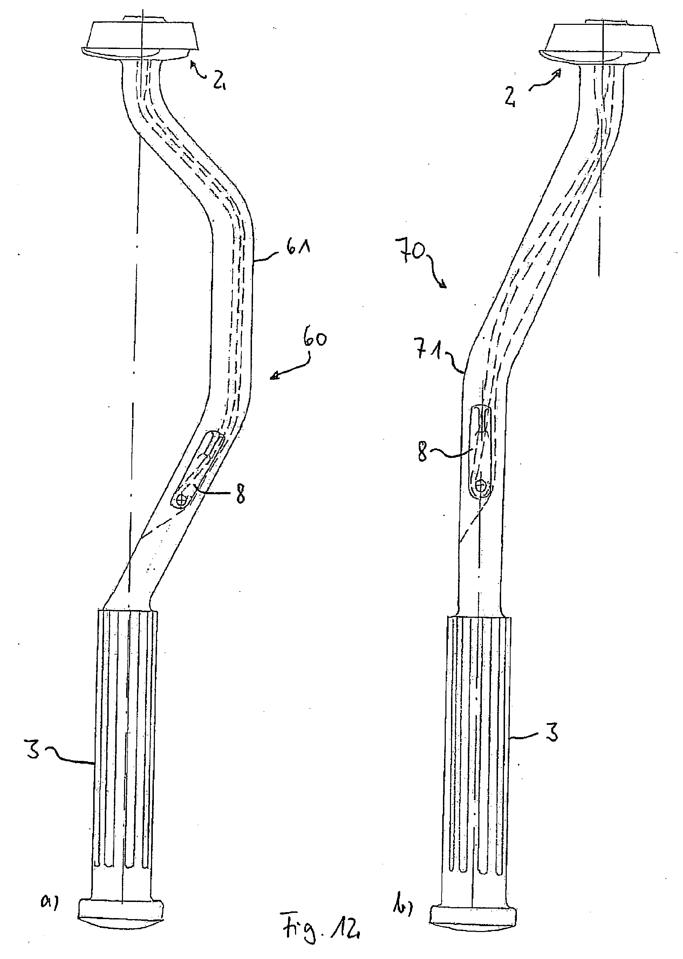

- Fig. 12: in zwei Darstellungen a und b zwei weitere Varianten eines erfindungsgemäßen Werkzeuges mit gekröpften Schaft; und

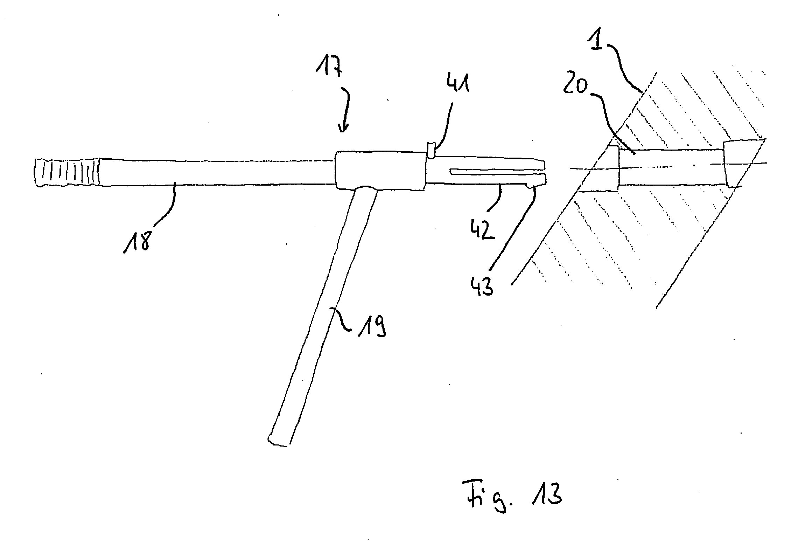

- Fig. 13: in einer Ausschnittsdarstellung die Gestaltung der Verbindung eines Peilinstrumentes mit dem erfindungsgemäßen Werkzeug an dessen Schaft.

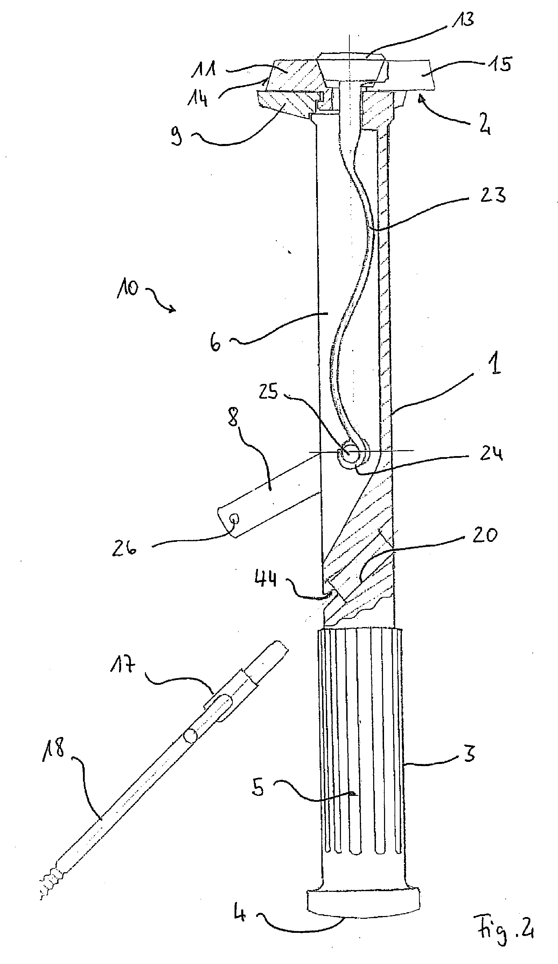

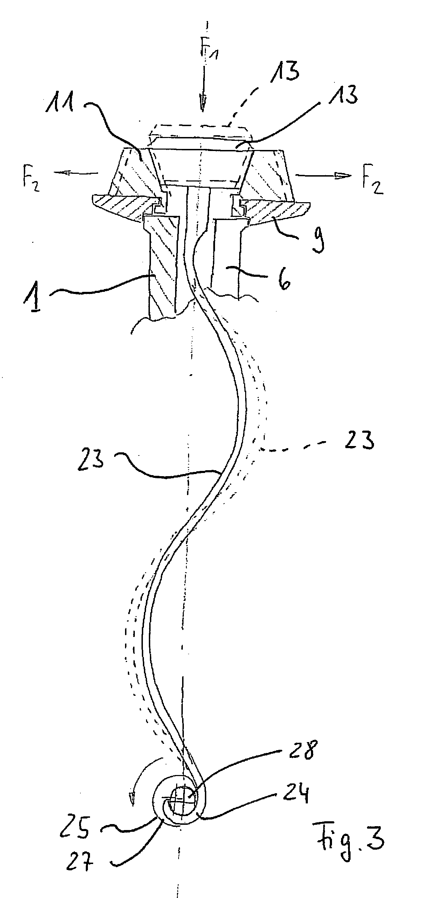

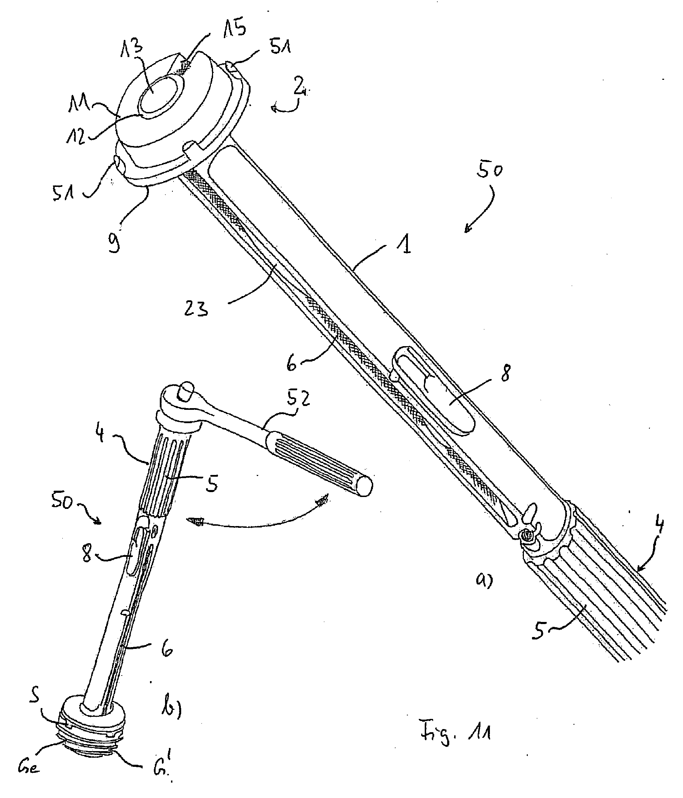

In den Figuren, bei denen es sich um schematische Darstellungen handelt, die keineswegs maßstabsgerecht sind und die lediglich der Erläuterung des Wirkprinzips und Aufbaus möglicher erfindungsgemäßer Ausführungsbeispiele dienen, sind unterschiedliche mögliche Ausführungsformen eines erfindungsgemäßen Werkzeuges gezeigt und dargestellt. Eine erste solche Ausführungsform ist in den Figuren 1 und 2 gezeigt, Detailansichten hierzu mit Erläuterungen, die auch für die nachfolgenden Ausführungsbeispiele herangezogen werden können, folgen in den Figuren 3 bis 10. In den Figuren 11 bzw. 12 sind weitere Ausführungsbeispiele dargestellt. Figur 13 zeigt eine Detaildarstellung der Anbindung eines optionalen Peilinstrumentes an ein erfindungsgemäßes Werkzeug.

Nachfolgend wird zunächst eine erste Ausführungsform eines erfindungsgemäßen Werkzeuges beschrieben, wie sie insbesondere in den Figuren 1 und 2 gezeigt ist mit weiteren Detaildarstellungen in den Figuren 3 bis 10.

Ein solches erstes Ausführungsbeispiel eines erfindungsgemäßen Werkzeuges 10 zum Setzen und/oder Entfernen von Gelenkkomponenten künstlicher Gelenkpfannen, wie es in den Figuren 1 und 2 dargestellt ist, weist einen langgestreckten und gradlinig verlaufenden Schaft 1 auf, der an einem ersten, distalen Ende einen Halteabschnitt 2 zum Festlegen einer einen Pfannenhohlraum aufweisenden Gelenkkomponente aufweist. Im Bereich des gegenüberliegenden zweiten, proximalen Endes ist an dem Schaft 1 ein Griffabschnitt 3 angeordnet. Am äußersten Ende des distalen Endes des Griffabschnittes 3 befindet sich eine Verdickung 4, die dem Einwirken auf das Werkzeug 10 mittels eines Schlaginstrumentes zum Einschlagen einer in dem Halteabschnitt 2 wie später nachfolgend erläutert gehaltenen Gelenkkomponente in den Knochen während einer Operation dient. Der Griffabschnitt 3 ist mit Längsriefen 5 strukturiert, die einer besseren Griffigkeit und mithin Handhabung dienen. Der Griffabschnitt 3 kann dabei mit einer für die Haptik und das Handling günstigen Oberfläche versehen sein, beispielsweise einer solchen aus Kunststoff. Der übrige Schaft ist ansonsten mit Vorteil aus einem Metall gebildet, beispielsweise Edelstahl.

Zu erkennen ist in Fig. 1 ferner, dass in dem Schaft 1 ein Längsschlitz 6 eingebracht ist, dessen Bedeutung später noch näher erläutert werden wird. In etwa über die gleiche Länge des Schaftes 1, wie der Längsschlitz 6 erstreckt sich eine Abflachung 7 entlang des Schaftes 1. Auf dieser sitzt ein Schwenkhebel 8, der in später näher zu erläuternder Weise mit einer Exzenterwelle verbunden ist. In dem Halteabschnitt 2 sind ferner zu erkennen eine Anlageplatte 9, ein auf dieser Anlageplatte 9 aufgelegtes elastisches Element 11 mit einer zentralen Öffnung 12 sowie ein in dieser zentralen Öffnung 12 sitzender Spannkörper 13. Das elastische Element 11 weist eine Höhe auf, entlang derer es eine umlaufende, an die Form der Innenkontur eines Pfannenhohlraumes einer Gelenkkomponente angepasste, hier konische, Außenfläche hat, die eine Klemmfläche 14 bildet. Ausgehend von der zentralen Öffnung 12 sind über die gesamte Höhe des elastischen Elementes radial verlaufende Schlitze 15 bzw. 16 eingebracht. Während der Schlitz 15 insgesamt bis nach außen durchgeführt ist, sind die beiden Schlitze 16 nur über einen Teil der radialen Strecke geführt, so dass im Bereich der Klemmefläche 14 eine Verbindung bestehen bleibt, das elastische Element 11 mithin einstückig gebildet ist. Die Schlitze 15 bzw. 16 sind in etwa gleichmäßig verteilt, d.h. jeweils um einen Winkel von etwa 120° zueinander beabstandet.

Schließlich ist in der Fig. 1 noch ein Peilinstrument 17 mit zwei in einem vorgegebenen Winkel fest miteinander verbundenen Peilstäben 18, 19 zu erkennen, welches optional in vorgegebener Ausrichtung in eine hierfür vorgesehene Aufnahmeöffnung 20 in dem Schaft 1 des Werkzeuges 10 eingesetzt werden kann. Dieses Peilinstrument 17 dient in an sich bekannter Weise dem Operateur als Hilfsmittel für die Ausrichtung der in dem Halteabschnitt 2 angeordneten bzw. an diesem festgelegten Gelenkkomponente.

Schließlich sind noch zwei im Schwenkbereich des Hebels 8 unterhalb desselben in der Abflachung 7 eingebrachte nutenartige Vertiefungen 21 bzw. 22 zu erkennen, die in später noch näher erläuterter Weise Bestandteile von Anschlägen für den Schwenkhebel 8 bilden.

In Fig. 2 ist in einer teilweise weg geschnittenen Darstellung das Werkzeug 10 gemäß Fig. 1 noch einmal in etwas anderer Positionierung gezeigt. Dabei ist insbesondere sehr gut der Längsschlitz 6 in dem Schaft 1 zu erkennen, der insgesamt eine Aufnahme bildet, in der eine Federzugstange 23 angeordnet ist. Die Federzugstange 23 bildet ein Zugmittel und ist mit einem ersten, proximalen Ende mit dem Spannkörper 13 verbunden, kann mit diesem einstückig gebildet sein. Zusammen mit dem Spannkörper 13 bildet die Federzugstange 23 ein Spreizmittel.

Mit einem zweiten, maximalen Ende ist die Federzugstange 23, die S-förmig gebogen bzw. gekrümmt verläuft, mit einer durch hakenförmiges Umbiegen dieses Endes entstandenen offenen Öse 24 auf einer Welle 25 angeordnet. Diese Welle 25 ist fest mit dem Schwenkhebel 8 verbunden und kann durch Verschwenken des Schwenkhebels 8 in Rotation versetzt werden. Dabei ist die Welle 25, wie aus nachfolgenden Darstellungen und im Zusammenhang mit Beschreibungen derselben noch deutlicher werden wird, als Exzenterwelle gebildet, wobei ein mittlerer Abschnitt, auf dem die offene Öse 24 angeordnet ist, exzentrisch zu der Drehlagerung in seitlichen Abschnitten gebildet ist. Dadurch wird eine Rotation der Welle, hervorgerufen durch Verschwenken des Schwenkhebels 8 in eine in Längsrichtung des Schaftes 1 wirkende Zug- bzw. Druckbewegung umgesetzt, so dass damit die Federzugstange 23 in Richtung ihres distalen bzw. proximalen Endes bewegt wird, mit dieser auch der am distalen Ende der Federzugstange 23 angeordnete Spannkörper.

Zu erkennen ist in dieser Darstellung ferner, dass der Spannkörper 13 ausgehend von einem breiten Durchmesser am distalen Ende hin zum proximalen Ende verjüngt verläuft, hier insbesondere konisch verjüngt. Die Kontur der zentralen Öffnung, in welcher der Spannkörper 13 sitzt, ist korrespondierend ausgebildet. Dadurch wird das elastische Element 11 dann, wenn der Spannkörper 13 in Richtung des proximalen Endes des Schaftes 1 gezogen wird, gespreizt. Dabei dient die Anschlagplatte 9 als Gegenanschlag, sodass das elastische Element 11 nicht in Richtung des proximalen Endes ausweichen kann, sondern durch eine Relativverschiebung des Spannkörpers 13 in Längsrichtung des Schaftes 1 in der zentralen Öffnung in dem elastischen Element 11 die Spreizkraft übertragen wird. Dies wird noch einmal deutlicher beim Betrachten der Fig. 3, die weiter unten stehend eingehender beschrieben und erläutert wird.

In Fig. 2 ist weiterhin noch zu erkennen ein auf der hier gezeigten Unterseite des Schwenkhebels 8 angeordneter Anschlagstift 26, der beim Verschwenken des Schwenkhebels 8 in die nutenartigen Vertiefungen 21 bzw. 22 eingreift und dort zusammen mit dem Rand dieser Vertiefungen jeweils einen Anschlag für die Schwenkbewegung des Schwenkhebels 8 bildet.

In Fig. 3 ist in einer Detaildarstellung des distalen Endes des Schaftes 1 des Werkzeuges noch einmal die Funktionsweise des Spannmittels gezeigt und dargestellt. Gezeigt ist das Spannmittel gebildet durch die Federzugstange 23 mit an deren distalen Ende angeordnetem Spannkörper 13 in zwei Stellungen, einmal in durchgezogener Linie in einer Spannstellung, einmal in gestrichelter Linie in einer gelösten Stellung. Gut zu erkennen ist, dass in der Spannstellung der Spannkörper 13 gegenüber der gelösten Stellung tiefer in die zentrale Öffnung des elastischen Elementes 11 eingedrungen ist, so dass durch die Keilwirkung ein Spreizen dieses elastischen Elementes 11, welches in Längsrichtung durch die Anlageplatte 9 gelagert ist, erfolgt. Wenn hingegen das Spannmittel gebildet durch die Federzugstange 23 und den Spannkörper 13 in einer entspannten Stellung befindlich ist, ragt der Spannkörper 13 weiter aus der zentralen Öffnung in dem elastischen Element 11 heraus, so dass dieses sich aufgrund der eigenen Elastizität und der dadurch hervorgerufenen Rückstellkräfte aus der geweiteten, in Umfangsrichtung ausgedehnten Position wieder zurückbewegt in eine entspannte Position, in welcher es einen geringeren Umfang einnimmt. Angedeutet sind diese Verhältnisse auch noch einmal durch Bezeichnung der Kräfte F1, der durch Spannen des Spannmittels aufgebrachten Zugkraft, sowie F2, der nach außen wirkenden Spreiz- bzw. Klemmkräfte des elastischen Elementes. Auch gut zu erkennen ist hierbei, dass beim Spannen, also beim Bewegen der Federzugstange 23 in Richtung des proximalen Endes eine Verformung des S eintritt, hier mithin eine elastische Aufnahme von Zugkraft F1 erfolgt. Diese Federwirkung in Längsrichtung der Federzugstange 23, die ansonsten in der Querrichtung vergleichsweise starr gebildet ist, hat verschiedene Wirkungen und Gründe, von denen eine eine Begrenzung der auf das elastische Element 11 aufgebrachte, nach außen in radialer Richtung wirkenden Spannkraft F2 mit sich bringt. Dies soll einer Beschädigung des elastischen Elementes, aber insbesondere auch einer Beschädigung der damit in nachfolgend noch näher zu erläuternden Weise aufgespannten und festgehaltenen Gelenkkomponente vorbeugen.

Zu erkennen ist in dieser Fig. 3 auch, wodurch diese Längsverlagerung der Federzugstange 23 und damit des Spannmittels hervorgerufen wird, nämlich durch die Welle 25, bei der es sich um eine Exzenterwelle handelt. In einem Abschnitt 27, in welchem die Exzenterwelle gelagert ist, hat diese einen anderen Rotationsmittelpunkt als in einem exzentrisch angeordneten, mittleren Abschnitt 28, an dem das proximale Ende der Federzugstange 23 mit seiner offenen Öse 24 festgelegt ist. Dadurch wird bei einer Rotation der Welle 25 die Lage des Abschnittes 28 in Längsrichtung des Schaftes 1 verschoben, es ergibt sich durch entsprechende Rotation der Welle 25, ausgelöst durch Verdrehen des Hebels 8 eine Bewegung des Zugmittels, gebildet durch Federzugstange 23 und Spannkörper 13, in Längsrichtung des Schaftes 1 in Richtung des proximalen bzw. distalen Endes zum Spannen bzw. Entspannen des elastischen Elementes 11 in radialer Richtung.

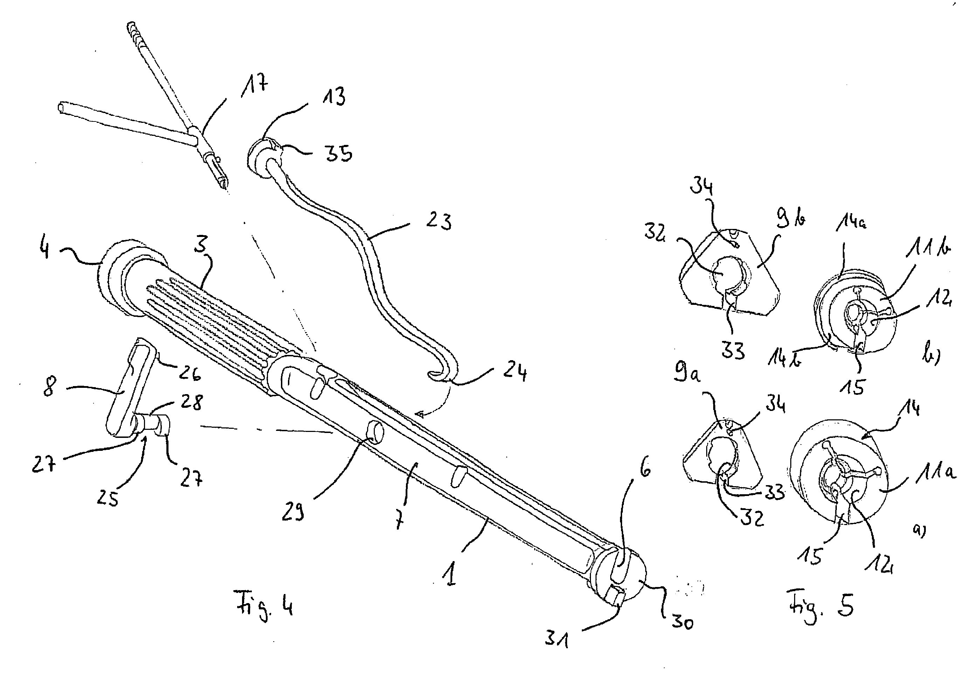

In Fig. 4 ist das erfindungsgemäße Werkzeug 10 in Teilen in einer Explosionsdarstellung zu erkennen. In dieser Darstellung fehlen lediglich die Anlageplatte und das elastische Element, die zusammen das Klemmmittel bilden und in Fig. 5 näher dargestellt sind, auf die hier ebenfalls Bezug genommen wird.

Diese Darstellung zeigt insbesondere die Zerlegbarkeit des Werkzeuges in wenige Teile, wobei dieses Zerlegen mit wenigen Handgriffen erfolgen kann. Ferner ist noch einmal gut der Schwenkhebel 8 zu erkennen, wie er fest mit der Welle 25 verbunden ist, die mit ihren Abschnitten 27 in der entsprechenden Öffnung 29 im Schaft 1 sitzen und um diese gedreht wird und die einen zwischen diesen Abschnitten 27 gelegenen, exzentrisch angeordneten Abschnitt 28 aufweist, auf dem die Federzugstange 23 mit der offenen Öse 24 sitzt. Das erfindungsgemäße Werkzeug kann sehr einfach in wenige Teile zerlegt werden, bestehend aus dem Schaft 1 mit fest angeformtem Griffstück 3, dem Schwenkhebel 8 mit angeformter Welle 25, dem Spannmittel in Form der Federzugstange 23 mit angeformtem Spannkörper 13 sowie eine in der Fig. 4 nicht dargestellten, in Fig. 5 in unterschiedlichen Varianten gezeigten Kombination aus Anlageplatte 9 und elastischem Element 11, die zusammen das Klemmmittel bilden. Optional kann sehr einfach das Peilinstrument 17 angeordnet und verrastet werden (siehe dazu später die Beschreibung zu Fig. 13).

Das Zusammenfügen bzw. Zerlegen des erfindungsgemäßen Werkzeuges geschieht sehr einfach. Für den Zusammenbau wird zunächst der Schwenkhebel 8 mit seiner Welle 25 in die Öffnung 29 eingeschoben. Sodann wird die Federzugstange 23 in einer wie in Fig. 4 gezeigten Stellung, in der der Spannkörper 13 in Richtung des proximalen Endes des Schaftes 1 weist, mit der offenen Öse 24 über den mittleren Abschnitt 28 der Welle 25 geführt. Anschließend wird in nachfolgend noch zu beschreibender Weise das Klemmmittel bestehend aus der Anlageplatte 9 und dem elastischen Element 11 zusammengefügt, über die Federzugstange 23 geführt, und die Federzugstange 23 wird umgeschwenkt mit ihrem Spannkörper 13 in Richtung des distalen Endes des Schaftes 1, bis die Anlageplatte 9 mit dem elastischen Element 11 zusammen auf die glatte Stirnfläche 30 des Schaftes 1 am distalen Ende aufgleitet. Für die korrekte Ausrichtung des Klemmmittels bestehend aus der Anlageplatte 9 und dem elastischen Element 11 auf der Stirnfläche 30 ist dort eine Nase 31 vorgesehen, die in nachfolgend noch näher zu beschreibender Weise mit der Anlageplatte 9 und dem elastischen Element 11 zusammenwirkt.

In den Figuren 5a und 5b sind zwei Kombinationen aus aufeinander abgestimmten Anlageplatten 9a bzw. 9b und elastischen Elementen 11a bzw. 11b gezeigt, aus denen unterschiedlich dimensionierte und geometrisch geformte Klemmmittel gebildet werden können, zum Verbinden mit unterschiedlichen Typen und Formen von Gelenkkomponenten. Dabei ist das in Fig. 5a gezeigte Paar im Wesentlichen entsprechend demjenigen, welches bei dem Werkzeug 10 gemäß Fig. 1 bzw. 2 eingesetzt ist. Es weist eine glattwandige und konisch verlaufende Klemmfläche 14 auf. Bei dem elastischen Element gemäß Fig. 5b ist eine Ringwulst 14a am proximalen Ende der Klemmfläche 14b ausgebildet und steht über diese vor. Diese Ringwulst 14a wirkt in später noch näher zu beschreibender Weise beim Verbinden mit einer Gelenkkomponente mit einem im Pfannenhohlraum derselben ausgebildeten Hinterschnitt zusammen, sorgt somit nicht nur für einen Verklemmen derselben, sondern auch für eine formschlüssige Verbindung und einen entsprechenden Halt.

In beiden Varianten der in Fig. 5 gezeigten Klemmelemente 11a bzw. 11b ist gut die zentrale Öffnung 12 zu erkennen, die für beide Klemmelemente gleich gebildet konisch sich verjüngend verläuft. Die Tatsache, dass diese zentrale Öffnung 12 in beiden Klemmelementen 11a und 11b gleich gebildet ist, ist insofern von Bedeutung, als dass diese elastischen Elemente 11a und 11b mit ein und demselben Spannmittel, also der Federzugstange 23 mit daran angeordnetem Spannkörper 23 verwendet werden kann. Selbstverständlich wäre es auch möglich, für unterschiedliche Klemmmittel unterschiedliche Spannmittel vorzusehen, was aber das erfindungsgemäße Werkzeug verkomplizieren würde, insoweit nicht bevorzugt ist.

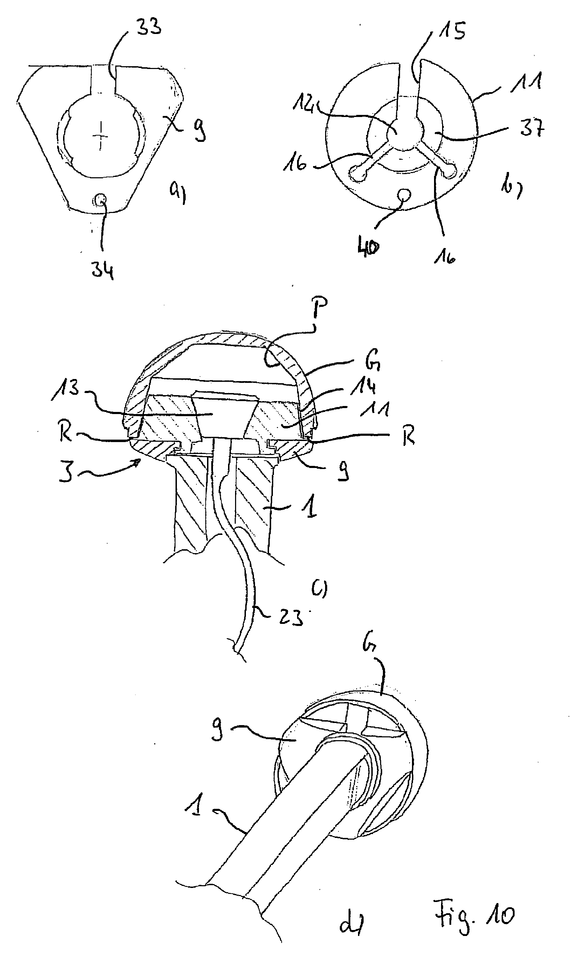

Auch in Fig. 5 zu erkennen ist, dass in den Anlageplatten 9a, 9b ebenfalls eine zentrale Öffnung 32 jeweils ausgebildet ist, die über einen Schlitz 33 jeweils nach außerhalb der Anlageplatte 9a bzw. 9b geöffnet ist. Ferner ist zu erkennen, dass die Anlageplatten 9a und 9b im Wesentlichen dreieckförmige Grundrisse aufweisen mit abgerundeten Ecken. Dieser dreieckige Grundriss wird in seiner Bedeutung nachfolgend noch näher erläutert werden anhand der Beschreibung zu Fig. 10.

Auch gezeigt sind Ausrichtstifte 34, die auf den Anlageplatten 9a bzw. 9b auf der Seite sich ausgehend von der Oberfläche erhaben erstrecken, auf der die elastischen Elemente 11a bzw. 11b jeweils mit ihrer Unterseite ruhen. Die elastischen Elemente 11a, 11b haben auf ihrer Unterseite, hier nicht zu erkennen, entsprechende Aufnahmeöffnungen, in die der Ausrichtstift 34 jeweils eingreift, so dass beim Zusammenfügen dieser Elemente eine eindeutige Ausrichtung vorgegeben ist. Diese eindeutige Ausrichtung erfolgt dabei so, dass der jeweilige durchgehende Schlitz 15 in dem elastischen Element 11a bzw. 11b mit dem Schlitz 33 in der Anlageplatte 9 a bzw. 9b fluchtet. Mit dieser Kombination aus zueinander fluchtenden Schlitzen 15, 33 kann dann eine Kombination aus Anlageplatte 9a bzw. 9b und elastischem Element 1 1 a bzw. 11 b über die Federzugstange 23 geführt werden, der Spannkörper 13 kann sodann in die zentrale Öffnung 12 des elastischen Elementes 11a bzw. 11b eingeführt werden. Wie in Fig. 4 zu erkennen ist, verfügt der Spannkörper 13 über einen aus seinem ansonsten sich konisch verjüngenden Umfang hervortretenden Ausrichtsteg 35, der dimensioniert und beschaffen ist, über ein geringes Maß in den Schlitz 15 des elastischen Elementes 11a bzw. 11b einzugreifen, so dass auch hier für eine eindeutige Ausrichtung gesorgt ist. Eine zusätzliche Ausrichthilfe gibt an der Stirnfläche 30 des Schaftes 1 die dort angeordnete Nase 31, die in den Schlitz 33 der Anlageplatte 9a bzw. 9b eingreift, je nach Ausgestaltung auch noch in den Schlitz 15 des elastischen Elementes 11a bzw. 11b eingreifen kann.

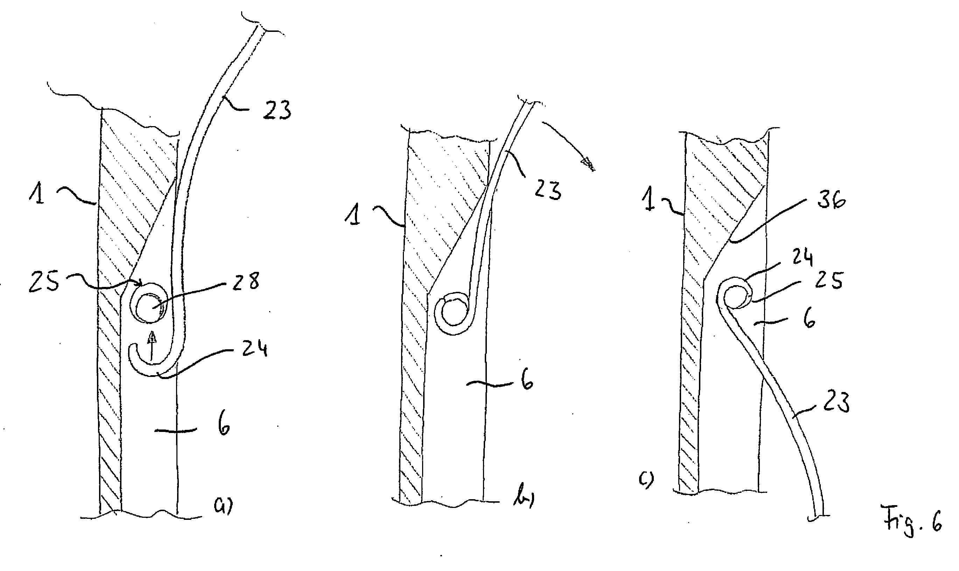

In den Figuren 6a bis c ist noch einmal in vergrößerter Darstellung die Montage der Federzugstange 23 durch Aufsetzen auf den mittleren Abschnitt 28 der Welle 25 erläutert. In der Fig. 6a ist gezeigt, wie die Federzugstange 23 mit in Richtung des proximalen Endes des Schaftes weisendem Spannkörper (hier nicht dargestellt) mit ihrner offenen Öse 24 in den Längsschlitz 1 6 vom distalen Ende des Schaftes her eingeführt und über den mittleren Abschnitt 28 der Welle 25 gelegt werden kann. Auf dieser Seite der Welle 25 und in dieser Ausrichtung ist im Inneren des Längsschlitzes 6 genügend Raum, um dieses vorzunehmen. In Fig. 6b ist dann angedeutet, wie die Federzugstange 23 verschwenkt wird in Richtung des distalen Endes des Schaftes 1. In Fig. 6c schließlich ist dargestellt, wie die Federzugstange 23 nahezu vollständig verschwenkt ist in Richtung des distalen Endes des Schaftes 1. Der besondere Verlauf der Begrenzungslinie des Längsschlitzes 6 entlang der Schräge 36, wo diese in unmittelbarer Nähe zu der offenen Öse 24 liegt, verhindert, dass in dieser Stellung sich die Federzugstange 23 von der Welle 25 lösen kann und schafft so eine einfache Vorverriegelung.

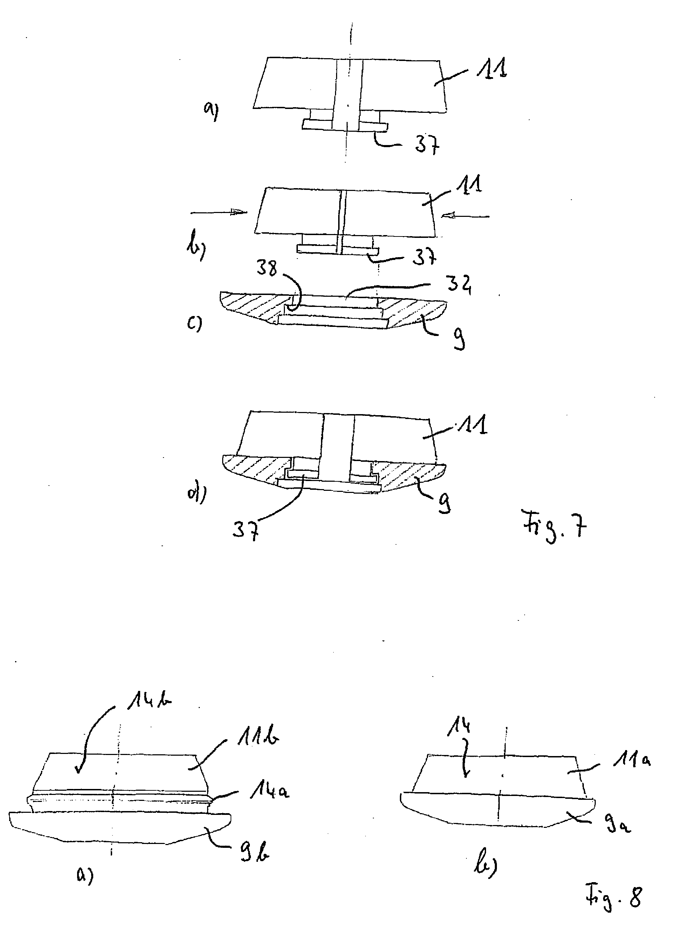

In Fig. 7 ist in vier unterschiedlichen Darstellungen noch einmal genauer gezeigt, wie das elastische Element 11 mit der Anlageplatte 9 verbunden wird und letztlich an dieser hält. Zu erkennen ist, dass das elastische Element 11 auf seiner im montierten Zustand dem proximalen Ende des Schaftes zugewandten Unterseite eine im Querschnitt T-förmige Verlängerung 37 aufweist (vgl. Figuren 7a und 7b insbesondere). Durch radiales Zusammendrücken des elastischen Elementes 11 aus seiner Ausgangsstellung gemäß Fig. 7a wird der Radius der T-förmigen Verlängerung 37 reduziert, wie dies in Fig. 7b angedeutet ist. In dieser Stellung kann das elastische Element 11 mit seiner T-förmigen Verlängerung 37 in die zentrale Öffnung 32 der Anlageplatte 9 (vgl. Fig. 7c) eingeführt werden. Löst man nun den Druck von dem elastischen Element 1 1 , entspannt sich dieses in radialer Richtung, und die T-förmige Verlängerung 37 rastet hinter einen Hinterschnitt 38 in der zentralen Öffnung 32 der Anlageplatte 9, wodurch die in Fig. 7d dargestellte Kombination aus Anlageplatte 9 und elastischem Element 11, die nun miteinander verbunden bzw. verrastet sind, entsteht, also das Klemmmittel.

In den Figuren 8a und b sind in einer Darstellung von der Seite noch einmal unterschiedliche Kombinationen aus einer Anlageplatte 9a und einem elastischen Element 11a (vgl. Fig. 8b, vergleichbar der Darstellung in Fig. 5a) bzw. aus Anlageplatte 9b und elastischem Element 1 1 b (vgl. Fig. 8a, in ähnlicher Darstellung wie in Fig. 5b) gezeigt. Zu erkennen ist hier noch einmal gut die umlaufende Ringwulst 14a an der Klemmfläche 14b des elastischen Elementes 1 1 b, im Gegensatz zu der glatt und allein konisch verlaufenden Klemmfläche 14 des elastischen Elementes 11a.

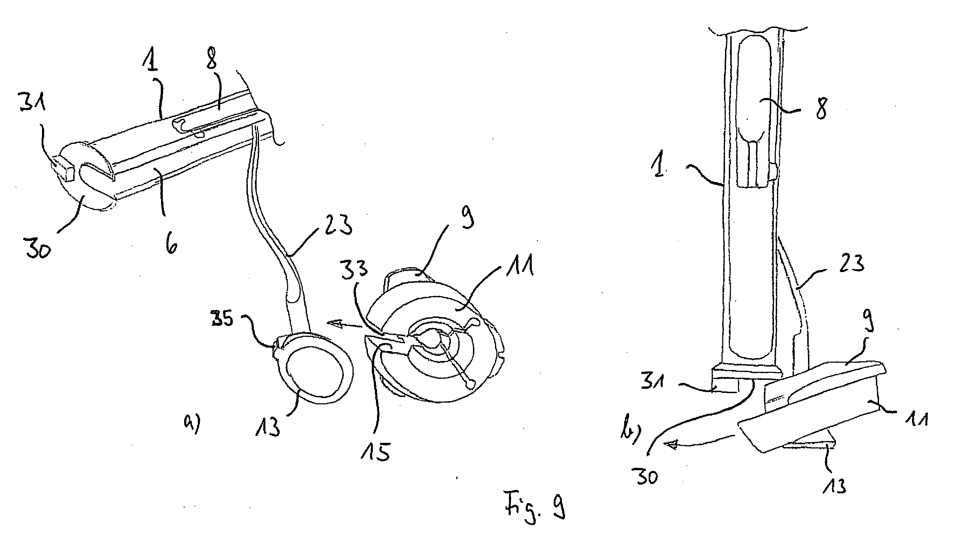

In den Figuren 9a und b ist noch einmal im Detail gezeigt, wie die Montage des aus Anlageplatte 9 und elastischem Element 11 gebildeten Klemmmittels am durch die Federzugstange 23 und den Spannkörper 13 verkörperten Spannmittel erfolgt und anschließend die korrekte Ausrichtung und Positionierung an der Stirnfläche 30 des Schaftes 1 zum vollständigen Zusammenbau des Werkzeuges.

Zunächst wird, wie dies in Fig. 9a dargestellt ist, die wie vorstehend beschrieben zusammengefügte Kombination aus Anlageplatte 9 und elastischem Element 11 mit den in Flucht ausgerichteten Schlitzen 32 und 15 in der wie durch den Pfeil angedeuteten Aufschubrichtung über die Federzugstange 23 geschoben. Durch den Ausrichtsteg 35 an dem Spannkörper 13 wird die richtige Ausrichtung des Klemmmittels, gebildet aus der Anlageplatte 9 und dem elastischen Element 11 vorgegeben. Nun kann die Federzugstange 23 weiter in Richtung des Längsschlitzes 6 verschwenkt werden, so dass die in Fig. 9b gezeigte Situation entsteht. Dort gleitet die Anlageplatte 9 über eine Außenkante der Stirnfläche 30 und rutscht so plan auf die Stirnfläche 30 auf, wobei der Ausrichtsteg 35 in den Schlitz 33 in der Anlageplatte 9 eingreift zur Ausrichtung des Klemmmittels. Dabei ist der Schwenkhebel 8 so verschwenkt, dass die Federzugstange 23 in ihrer maximal in Richtung des distalen Endes des Schaftes 1 verlagerten Stellung befindlich ist. Auch in dieser Stellung, so sind die Bemaßungen der Elemente aufeinander abgestimmt, ist die Situation so, dass ein Verschieben der Anlageplatte 9 über die Kante der Stirnfläche 30 des Schaftes 1 nicht einfach so und mit Spiel möglich ist, sondern lediglich unter Aufbringen einer gewissen Spannkraft. Diese Spannkraft, die zugleich eine Haltekraft darstellt, welche die Kombination aus Anlageplatte 9 und elastischem Element 11 in der Montageposition am distalen Ende des Schaftes 1, also auf der dortigen Stirnfläche 30, hält, wird gebildet durch das elastische Element 11 selbst, welches beim über die Kante Schieben der Anlageplatte 9 durch ein Eindringen des Spannkörpers 13 in die zentrale Öffnung gespreizt wird gegen eine Rückstellkraft, die das elastische Element 11 selbst aufbringt, und/oder durch eine Federkraft der Federzustange 23, die bei diesem Vorgang in Längsrichtung gestreckt wird entgegen ihrer Federwirkung. So wird also beim Aufschieben der Anlageplatte 9 mit darauf montiertem elastischen Element über die Kante auf die Stirnfläche 30 eine Federkraft aufgewandt, bis die Anlageplatte 9 über einen Totpunkt hinübergeschoben ist und das System dann in die Endposition schnappt, in der die Anlageplatte 9 auf der Stirnfläche 30 aufliegt.

Ein Ablösen des Klemmmittels kann dann durch über die Kante Schieben der Anlageplatte 9 mit darauf montiertem elastischen Element 11 in umgekehrter Richtung und wiederum gegen eine Rückstellkraft erfolgen, die in gleicher Weise durch das elastische Element 11 selbst, in das der Spannkörper 13 eindringt und dieses gegen Rückstellkraft spreizt, und/oder durch die Federzugstange 23 aufgebracht wird. Um hier den Zusammenbau bzw. das Trennen zu vereinfachen, kann an derjenigen Stelle, an welcher die Anlageplatte 9 über die Kante der Stirnfläche 30 gleitet, eine entsprechende Auflaufschräge ausgebildet sein. Beim Zusammenbau des Werkzeuges, auch dies lässt sich aus Fig. 9 ablesen, gerät die Federzugstange 23 in der Endposition in das Innere des Schaftes 1, genau genommen in die durch den Längsschlitz 6 gebildete Aufnahme, so dass sie während des Gebrauches nicht störend seitlich über den Schaft 1 übersteht.

In Fig. 10 sind in verschiedenen Ansichten a bis d noch weitere Darstellungen gegeben. In Fig. 10a ist noch einmal eine Ansicht einer Anlageplatte 9 von oben dargestellt mit dem Ausrichtstift 34. Zu erkennen sind auch der Schlitz 33 sowie die erneut dreieckige Grundform. In Fig. 10b ist in einer Ansicht von unten her, also vom proximalen Ende das elastische Element 11 gezeigt, wobei hier eine Ausrichtöffnung 40 zu erkennen ist, in die der Ausrichtstift 34 im zusammengesetzten Zustand eingreift, um so die richtige Orientierung des Klemmelementes 11 gegenüber der Anlageplatte 9 sicherzustellen.

In Fig. 10 ist in einer Ausschnittsdarstellung im Schnitt zu erkennen, wie an dem erfindungsgemäßen Werkzeug eine Gelenkkomponente G angeordnet ist. Bei der dort gezeigten Gelenkkomponente G handelt es sich um den Basiskörper einer künstlichen Gelenkpfanne eines Hüftgelenkes, der in seinem Inneren einen Pfannenhohlraum P aufweist, in welchen zum Aufbau des künstlichen Gelenkes auch ein Einsatz eingesetzt wird, welcher die eigentliche Gelenkpfanne beinhaltet. Zum Setzen, aber auch zum Entfernen dieser Gelenkkomponente G wird mit dem erfindungsgemäßen Werkzeug 1 diese am Halteabschnitt 2 festgelegt. Dazu werden auf diese Gelenkkomponente G abgestimmte Haltemittel verwendet, gebildet aus einer Anlageplatte 9 und einem elastischen Element 11. Gut zu erkennen ist hier, wie die Gelenkkomponente G mit ihrem Rand R auf der Anlageplatte 9 aufliegt. Von Innen wird durch wie oben beschriebenes Aufbringen von Spreiz- bzw. Spannkräften mittels des Niederziehens des Spannkörpers 13 das elastische Element 11 mit seiner Klemmfläche 14 gegen die Innenwand des Pfannenhohlraumes geklemmt, die Gelenkkomponente G wird so fixiert und verankert. Nun kann die so fixierte und ausgerichtete Gelenkkomponente G unter der Operation gesetzt werden, beispielsweise durch Einschlagen mit einem Werkzeug 10, wie vorstehend beschrieben. Alternativ ist es auch möglich, eine implantierte Gelenkkomponente G mit einem erfindungsgemäßen Werkzeug zu entfernen, indem der Halteabschnitt 3 entsprechend in die implantierte Gelenkkomponente G in deren Pfannenhohlraum P eingebracht und dort in der beschriebenen Weise verankert und verklemmt und die Gelenkkomponente dann aus dem Knochen herausgezogen wird.

Aus Fig. 10d ist in einer Ansicht deutlich, welchen Vorteil die im Wesentlichen dreieckige Ausgestaltung der Anlageplatte 9 mit sich bringt. Dadurch sind nämlich einzelne Abschnitte des Umfanges der Gelenkkomponente G freistehend und für den Operateur sichtbar, so dass er eine bessere Übersicht behält und die Gelenkkomponente G in der Operation besser setzen bzw. auch entfernen kann. Hierzu ist nicht unbedingt eine dreieckige Grundform der Anlageplatte 9 erforderlich, es ist lediglich wichtig, dass diese zumindest an einer Stelle gegenüber der Außenkontur einer aufzulagernden Gelenkkomponente G zurückgesetzt ist, so dass sie diese bei aufgesetzter Gelenkkomponente G frei lässt und einen Blick darauf gestattet.

In Fig. 11 ist in zwei unterschiedlichen Ansichten a und b eine alternative Variante für ein erfindungsgemäßes Werkzeug 50 gezeigt. Dieses entspricht im grundsätzlichen Aufbau im Wesentlichen dem Aufbau, wie er oben in Bezug auf das Werkzeug 10 beschrieben ist. Der Unterschied besteht im Wesentlichen darin, dass dieses Instrument zum Handhaben von einzuschraubenden Gelenkkomponenten konzipiert ist. Hierzu sind auf der dem distalen Ende zugewandten Oberseite der Anlageplatte 9 nach oben weisende Halbzapfen 51 vorgesehen, die in entsprechende Gegenstrukturen S einer Gelenkkomponente G' eingreifen, um ein Drehmoment sicher übertragen zu können. Die Gelenkkomponente G' ist auf ihrer Außenseite mit Gewindezügen Ge versehen, mit denen sie in den Knochen eingreifen kann. Es handelt sich hierbei also um eine einschraubbare Gelenkkomponente. An seinem proximalen Ende weist das Werkzeug 50 eine Struktur zum Angreifen eines Schraubhebels 52 auf, der beispielsweise eine Ratsche sein kann. Es dürfte einleuchten, dass mit dem erfindungsgemäßen Werkzeug 50 Gelenkkomponenten G' ebenso gut aus einer Implantatposition im Knochen herausgeschraubt werden können, wie sie in diesen eingeschraubt, also gesetzt werden können.

Die Figuren 12a und b zeigen weitere Varianten erfindungsgemäßer Werkzeuge 60 bzw. 70, die bei ansonsten identischer Funktionalität und gleichartigem Aufbau abweichende Schaftformen aufweisen. So ist das Werkzeug 60 mit einem doppelt gekröpften Schaft 61 versehen, der jedoch eine durchgehende Achsausrichtung zwischen dem Griffstück 3 und dem Halteabschnitt 2 beibehält. In Fig. 12b ist ein ebenfalls doppelt gekröpfter Schaft 71 gezeigt, der einen parallelen Achsversatz zwischen der Längsachse des Werkzeuges im Bereich des Griffabschnittes 3 und der Längsachse des Werkzeuges im Bereich des Halteabschnittes 2 bewirkt. Diese Sonderformen sind bei unterschiedlichen besonderen Gegebenheiten und Operationstechniken von Vorteil.

Insgesamt können auch weitere Variationen von Schaftformen und Formen der Klemmmittel gewählt werden, wobei mit der Erfindung auch ein Satz zum Bilden eines bedarfsgerechten Werkzeuges offenbart ist, welcher Satz einen oder mehrere Schäfte aufweisen kann und insbesondere wenigstens zwei unterschiedliche Klemmmittel, d.h. für die jeweiligen Gelenkkomponenten angepasste Kombinationen aus z.B. Anlageplatten und zugehörigen elastischen Elementen. So kann mit einem begrenzten Umfang an Werkzeugteilen eine Vielzahl von unterschiedlichen Gelenkkomponenten in den unterschiedlichsten Situationen gehandhabt werden, was die Anzahl der anzuschaffenden Werkzeuge und damit die Kosten reduziert. Vor dem Hintergrund, dass das erfindungsgemäße Werkzeug einfach zu zerlegen und zu reinigen ist, wobei insbesondere die Art der Verbindung des Klemmmittels mit dem Spannmittel, wie sie anhand der Fig. 9 beschrieben ist, hervorsticht, ist das Set und ist ein erfindungsgemäßes Werkzeug einfach in der Handhabung, es ist insbesondere gut und leicht zu reinigen und zu sterilisieren.

In Fig. 13 ist noch einmal in vergrößerter Darstellung das als optionales Zubehör für ein erfindungsgemäßes Werkzeug beistellbare Peilinstrument 17 gezeigt mit den beiden Peilstäben 18 und 19. Insbesondere ist hier dargestellt, in welcher Weise die Festlegung des Peilinstrumentes 17 in der Aufnahmeöffnung 20 in dem Schaft 1 des Werkzeuges erfolgt. Hierzu hat das Peilinstrument 17 ein Rastende 42, welches einen Längsschlitz aufweist, so dass die beiden einander gegenüberliegenden Schenkel des Rastendes 42 aufeinender zu bewegbar sind. Am in der Fig. 13 äußerst rechts gezeigten Ende des Rastendes 42 ist ein Rastvorsprung 43 angeordnet, der bei in die Aufnahmeöffnung 20 eingeschobenem Rastende 42 hinter einem Hinterschnitt verrastet, das Peilinstrument 17 so hält. Durch Aufbringen einer entsprechenden rückwärts gerichteten Kraft, kann das Peilinstrument 17 gelöst werden. Ferner ist noch ein Stift 41 zu erkennen, der im Zusammenwirken mit einer schlitzförmigen Öffnung 44 (vgl. hierzu Fig. 2) die Ausrichtung des Peilinstrumentes vorgibt.

Die gezeigten Ausführungsbeispiele dienen lediglich der Erläuterung der Erfindung und sollen diese nicht beschränken. Sie haben jedoch noch einmal deutlich die vielfältigen Vorteile und Merkmale der Erfindung aufgezeigt.

### Bezugszeichenliste

- 1: Schaft
- 2: Halteabschnitt
- 3: Griffabschnitt
- 4: Verdickung
- 5: Längsriefen
- 6: Längsschlitz
- 7: Abflachung
- 8: Schwenkhebel
- 9: Anlageplatte
- 9a, b: Anlageplatte
- 10: Werkzeug
- 11: elastisches Element
- 11 a, b: elastisches Element
- 12: zentrale Öffnung
- 13: Spannkörper
- 14: Klemmfläche
- 14 a: Ringwulst
- 14 b: Klemmfläche
- 15: Schlitz
- 16: Schlitz
- 17: Peilinstrument
- 18: Peilstab
- 19: Peilstab
- 20: Aufnahmeöffnung
- 21: nutenartige Vertiefung
- 22: nutenartige Vertiefung
- 23: Federzugstange
- 24: offene Öse
- 25: Welle
- 26: Anschlagstift
- 27: Abschnitt
- 28: Abschnitt
- 29: Öffnung
- 30: Stirnfläche
- 31: Nase
- 32: zentrale Öffnung
- 33: Schlitz
- 34: Ausrichtstift
- 35: Ausrichtsteg
- 36: Schräge
- 37: T-förmige Verlängerung
- 38: Hinterschnitt
- 40: Ausrichtöffnung
- 41: Stift
- 42: Rastende
- 43: Rastvorsprung
- 44: schlitzförmige Öffnung
- 50: Werkzeug
- 51: Halbzapfen
- 52: Schraubhebel
- 60: Werkzeug
- 61: Schaft
- 70: Werkzeug
- 71: Schaft
- F₁: Zugkraft
- F₂: Spreizkraft
- G: Gelenkkomponente
- G': Gelenkkomponente
- Ge: Gewindezug
- P: Pfannenhohlraum
- R: Rand

## Patentansprüche

1. Werkzeug zum Setzen oder Entfernen von Gelenkkomponenten (G; G') künstlicher Gelenkpfannen, insbesondere für Hüftgelenkendoprothesen, mit einem langgestreckten Schaft (1 ; 61; 71), der an einem ersten, distalen Ende einen Halteabschnitt (2) zum Festlegen einer einen Pfannenhohlraum (P) aufweisenden Gelenkkomponente (G; G') und im Bereich eines zweiten, proximalen Endes einen Griffabschnitt (3) aufweist, wobei der Halteabschnitt (2) ein in den Pfannhohlraum (P) einführbares, radial nach außen spreizbares Klemmmittel (9, 11) sowie ein mit dem Klemmmittel zusammenwirkendes Spreizmittel (13, 23) zum nach außen Spreizen des Klemmmittels (9, 11) aufweist, wobei das Spreizmittel (13, 23) zum Spreizen bzw. Lösen des Klemmmittels (9, 11) in Richtung des Verlaufes des Schaftes (1; 61; 71) bewegbar ist und ein Zugmittel (23) aufweist, welches sich über zumindest einen Teil der Länge des Schaftes (1 ; 61; 71), vorzugsweise im Innern desselben, erstreckt, wobei das Zugmittel (23) in Längsrichtung des Schaftes (1; 61; 71) federelastisch gebildet ist, **dadurch gekennzeichnet, dass** das Zugmittel (23) als Federzugstange ausgebildet ist.

2. Werkzeug nach Anspruch 1, **dadurch gekennzeichnet, dass** die Federzugstange S-förmig oder doppelt S-förmig ist.

3. Werkzeug nach einem der vorhergehenden Ansprüche, **dadurch gekennzeichnet, dass** das Klemmmittel (9, 11) ein elastisches Element (11) aufweist mit einer zum distalen Ende hin sich erweiternd verlaufenden Öffnung (12) und dass das Spreizmittel (13, 23) einen in der Öffnung (12) des elastischen Elementes (11) sitzenden, in Richtung des proximalen Endes des Schaftes (1; 61; 71) bewegbaren Spannkörper (13) mit korrespondierender, sich in Richtung des proximalen Endes verjüngenden Außenkontur aufweist.

4. Werkzeug nach Anspruch 3, **dadurch gekennzeichnet, dass** das elastische Element (11) in einer Umfangsrichtung wenigstens an einer Position geschlitzt ist.

5. Werkzeug nach Anspruch einem der Ansprüche 3 oder 4, **dadurch gekennzeichnet, dass** das Klemmmittel (9, 11) eine am distalen Ende des Schaftes (1; 61; 71) angeordnete Anlageplatte (9) als Gegenlager für das elastische Element (11) und zur Anlage an einem Rand (R) der den Pfannenhohlraum (P) aufweisenden Gelenkkomponente (G; G') umfasst.

6. Werkzeug nach Anspruch 5, **dadurch gekennzeichnet, dass** die Anlageplatte (9) eine Öffnung (32) aufweist, in der das elastische Element (11) mit einem einen Hinterschnitt aufweisenden Halteabschnitt (37) lösbar festlegbar ist.

7. Werkzeug nach einem der Ansprüche 5 oder 6, **dadurch gekennzeichnet, dass** das Zugmittel (23) ein quer zur Längsrichtung des Schaftes (1; 61 ; 71) im Wesentlichen starres Element ist, welches um einen von dem distalen Ende in Richtung des proximalen Endes versetzt gelegenen Lagerpunkt (28) verschwenkbar an dem Schaft (1; 61; 71) festgelegt ist und an seinem distalen Ende die Anlageplatte (9) in einer Öffnung (32) durchragt, wobei das distale Ende des Schaftes (1; 61 ; 71) und das Spreizmittel (13, 23) und/oder das Klemmmittel (9, 11) so ausgebildet ist/sind, dass durch unter Überwindung einer Rückhaltekraft vorgenommenes Verschwenken des Zugmittels (23) die Anlageplatte (9) über das distale Ende des Schaftes (1; 61; 71) hinweggeschoben und so das Klemmmittel (9, 11) von dem distalen Ende des Schaftes (1 ; 61 ; 71) gelöst werden bzw. in umgekehrter Richtung an dem distalen Ende des Schaftes (1; 61; 71) angeordnet und festgelegt werden kann.

8. Werkzeug nach Anspruch 7, **gekennzeichnet durch** durchgehende Schlitze (33; 15) in der Anlageplatte (9) und dem elastischen Element (11), mittels derer die genannten Elemente über das Zugmittel (23) führbar und so auf dieses aufsetzbar bzw. von diesem lösbar sind.

9. Werkzeug nach einem der vorhergehenden Ansprüche, **gekennzeichnet durch** einen an dem Schaft (1; 61 ; 71) angeordneten Exzenterhebel (8, 25), auf dem ein proximales Ende des Zugmittels (23) in exzentrischer Lagerung angeordnet ist, zum Längsverschieben des Zugmittels (23) **durch** Betätigung des Exzenterhebels (8, 25).

10. Werkzeug nach Anspruch 9, **dadurch gekennzeichnet, dass** der Exzenterhebel (8, 25) einen Totpunkt aufweist, bei dessen Überschreiten er das Zugmittel (23) in einer Stellung verriegelt, in der das Spreizmittel (13, 23) das Klemmmittel (9, 11) radial nach außen spreizt.

11. Set zur bedarfsweisen Bildung eines Werkzeuges nach einem der vorhergehenden Ansprüche, umfassend einen Schaft (1; 61; 71) mit einem ein in Längsrichtung des Schaftes (1; 61; 71) federelastisches Zugmittel (23) in Form einer Federzugstange aufweisenden Spreizmittel (13, 23) und wenigstens zwei auswechselbar an dem distalen Ende des Schaftes (1; 61; 71) anordbare Klemmmittel (9, 11; 9a; 11a; 9b; 11b) unterschiedlicher Geometrie.

12. Set nach Anspruch 11, **dadurch gekennzeichnet, dass** es wenigstens zwei Schäfte (1 ; 61 ; 71) unterschiedlicher Form aufweist.

## Claims

1. Tool for placing or removing joint components (G; G') of artificial joint sockets, in particular for hip joint hypotheses, with an elongated shank (1; 61; 71), which comprises on a first distal end a retaining portion (2) for immobilising a joint component (G; G') having a socket cavity (P) and a grip portion (3) in the region of a second proximal end, wherein the retaining portion (2) has a clamping means (9, 11), which can be spread radially and outwardly and introduced into the socket cavity (P), as well as a spreading means (13, 23) co-operating with the clamping means for spreading said clamping means outwardly, wherein the spreading means (13, 23) is mobile for spreading or releasing the clamping means (9, 11) in the direction of the curve of the shank (1; 61; 71) and a traction means (23), which extends at least one portion of the length of the shank (1; 61; 71), preferably in the inside portion thereof, wherein the traction means (23) is designed resiliently in longitudinal direction of the shank (1; 61; 71), **characterised in that** the traction means (23) is designed as a spring tie-rod.

2. Tool according to claim 1, **characterised in that** the spring tie-rod is S-shaped or double S-shaped.

3. Tool according to any of the preceding claims, **characterised in that** the clamping means (9, 11) exhibits an elastic element (11) with an opening (12) widening towards the distal end and that the spreading means (13, 23) exhibits a clamping body (13) mobile in the direction of the proximal end of the shank (1; 61; 71) and situated in the opening (12) of the elastic element (12) with a matching external contour tapering in the direction of the proximal end.

4. Tool according to claim 3, **characterised in that** the elastic element (11) is slitted at least in one position around the periphery.

5. A tool according to one of claims 3 or 4, **characterised in that** the clamping means (9, 11) comprises a bearing plate (9) arranged on the distal end of the shank (1; 61; 71) acting as a counterbearing for the elastic element (11) and for installation on the edge (R) of the joint component (G; G') including the socket cavity (P).

6. Tool according to claim 5, **characterised in that** the bearing plate (9) comprises an opening (32) in which the elastic element (11) can be immobilised releasably with a retaining portion (37) having an undercut.

7. Tool according to one of claims 5 or 6, **characterised in that** the traction means (23) is a rigid element which is more or less crosswise to the longitudinal direction of the shank (1; 61; 71), which is immobilised pivotably on the shank (1; 61; 71) from a bearing point (28) offset from the distal end in the direction of the proximal end and the bearing plate (9) protrudes in an opening (32) at the distal and of the shank, wherein the distal end of the shank (1; 61; 71) and the spreading means (13, 23) and/or the clamping means (9, 11) are designed in such a way that by overcoming a retention force and with the initiated horizontal sweep of the traction means (23) the bearing plate (9) can be pushed away over the distal end of the shank (1; 61; 71) and thus the clamping means (9, 11) can be released from the distal end of the shank (1; 61; 71) or can be arranged and immobilised in reverse direction on the distal end of the shank (1; 61; 71).

8. Tool according to claim 7, **characterised by** continuous slits (33; 15) in the bearing plate (9) and the elastic element (11), by means of which the mentioned elements can be guided via the traction means (23) and thus be mounted thereon or released therefrom.

9. Tool according to any of the preceding claims, **characterised by** an eccentric lever (8, 25) arranged on the shank (1; 61; 71), on which a proximal end of the traction means (23) is arranged in an eccentric position, for longitudinal displacement of the traction means (23) by actuating the eccentric lever (8, 25).

10. Tool according to claim 9, **characterised in that** the eccentric lever (8, 25) has a dead centre which when being exceeded locks the traction means (23) in a position in which the spreading means (13, 23) spreads the clamping means (9, 11) radially and outwardly.

11. Set for assembling as required a tool according to any of the preceding claims, comprising a shank (1; 61; 71) having a spreading means (13, 23) in the form of a spring tie-rod, including a resilient traction means (23) in longitudinal direction of the shank (1; 61; 71) and at least two interchangeable clamping means (9, 11; 9a; 11 a; 9b; 11 b) of various geometry, which can be arranged on the distal end of the shank (1; 61; 71).

12. Set according to claim 11, **characterised in that** it contains at least two shanks (1; 61; 71) of various form.

## Revendications

1. Instrument pour la mise en place ou le retrait de composants articulaires (G; G') de cupules cotyloïdiennes artificielles, en particulier pour des prothèses articulaires de la hanche, comportant une tige longitudinale (1; 61; 71), présentant en une première extrémité distale une partie maintien (2) permettant la fixation d'un composant articulaire (G; G') comportant une cavité cotyloïdienne et une partie poignée (3) dans la zone d'une seconde partie proximale, où la partie maintien (2) présente un moyen de serrage (9, 11) pouvant se déployer radialement vers l'extérieur et pouvant s'introduire dans la cavité cotyloïdienne (P) de même qu'un moyen de déploiement (13, 23) coopérant avec le moyen de serrage pour déployer le moyen de serrage (9, 11) vers l'extérieur, où le moyen de déploiement (13, 23) est mobile pour déployer ou pour relâcher le moyen de serrage (9, 11) dans le sens de la courbe de la tige (1; 61; 71) et présente un moyen de traction (23), s'étendant sur au moins une partie de la longueur de la tige (1; 61; 71), de préférence à l'intérieur de celle-ci, où le moyen de traction (23) est conçu de manière résiliente dans l'axe longitudinal de la tige (1; 61; 71), **caractérisé en ce que** le moyen de traction (23) est conçu comme tige de traction à ressort.

2. Instrument selon la revendication 1, **caractérisée en ce que** la tige de traction à ressort est en forme de S ou en forme de double S.

3. Intrument selon l'une quelconque des revendications précédentes, **caractérisé en ce que** le moyen de serrage (9, 11) présente un élément élastique (11) comportant une ouverture (12) s'élargissant vers l'extrémité distale et que le moyen de déploiement (13, 23) présente un corps de serrage (13) mobile dans le sens de l'extrémité proximale de la tige (1; 61; 71) et situé dans l'ouverture (12) de l'élément élastique (11) avec un contour externe correspondant se rétrécissant dans le sens de l'extrémité proximale.

4. Instrument selon la revendication 3, **caractérisée en ce que** l'élément élastique (11) est fendu dans la direction circonférentielle au moins en un point.

5. Instrument selon l'une quelconque des revendications 3 ou 4, **caractérisé en ce que** le moyen de serrage (9, 11) présente une plaque d'appui (9) disposée à l'extrémité distale de la tige (1; 61; 71) comme contrepaliuer pour l'élément élastique (11) et pour reposer contre un bord (R) du composant articulaire (G; G') comportant la cavité cotyloïdienne (P).

6. Instrument selon la revendication 5, **caractérisé en ce que** la plaque d'appui (9) présente une ouverture (32) dans laquelle l'élément élastique (11) peut être immobilisé de façon amovible à l'aide d'une partie maintien (37) présentant une contre-dépouille.

7. Instrument selon l'une des revendications 5 ou 6, **caractérisé en ce que** le moyen de traction (23) est un élément plus ou moins rigide et perpendiculaire à la direction longitudinale de la tige (1; 61; 71), qui est immobilisé de manière pivotante sur la tige (1; 61; 71) par rapport à un point d'appui (28) décalé de l'extrémité distale dans la direction de l'extrémité proximale et la plaque d'appui (9) pénètre dans une ouverture (32) en l'extrémité distale de ladite tige, où l'extrémité distale de la tige (1; 61; 71) et le moyen de déploiement (13, 23) et/ou le moyen de serrage (9, 11) sont conçus de telle sorte qu'en s'opposant à la force de retenue et avec le pivotement initié du moyen de traction (23) la plaque d'appui (9) peut être repoussé sur la longueur de l'extrémité distale de la tige (1; 61; 71) et le moyen de serrage (9, 11) peut ainsi être libéré de l'extrémité distale de la tige (1; 61; 71) ou peut être disposé et immobilisé dans le sens inverse sur l'extrémité distale de la tige (1; 61; 71).

8. Instrument selon la revendication 7, **caractérisé par** des fentes continues (33; 15) dans la plaque d'appui (9) et l'élément élastique (11), à l'aide desquels les éléments cités peuvent être guidés sur la longueur du moyen de traction (23) et peuvent ainsi être montés sur celui-ci ou encore être libérés de celui-ci.

9. Intrument selon l'une quelconque des revendications précédentes, **caractérisé par** un levier excentré (8, 25) disposé sur la tige (1; 61; 71), sur lequel une extrémité proximale du moyen de traction (23) occupe une position excentrée, pour repousser longitudinalement le moyen de traction (23) en actionnant le levier excentré (8, 25).

10. Instrument selon la revendication 9, **caractérisé en ce que** le levier excentré (8, 25) présente un point mort qui en cas de dépassement verrouille le moyen de traction (23) dans une position dans laquelle le moyen de déploiement (13, 23) déploie le moyen de serrage (9, 11) radialement vers l'extérieur.

11. Ensemble de montage modulaire d'un instrument selon l'une des revendications précédentes, comprenant une tige (1; 61; 71) ayant un moyen de traction (23) résilient dans l'axe longitudinal de la tige (1; 61; 71) sous forme de moyen de déploiement (13, 23) comprenant une tige de traction à ressort et au moins deux moyens de serrage (9, 11; 9a; 11 a; 9b; 11 b) interchangeables et de géométrie diverse, pouvant être disposés à l'extrémité distale de la tige (1; 61; 71).

12. Ensemble selon la revendication 11 **caractérisée en ce qu'**il présente au moins deux tiges (1; 61; 71) de forme différente.
